# EUROPÄISCHE PATENTANMELDUNG

(11) **EP 0 885 616 A1**
(43) Veröffentlichungstag der Anmeldung: **23.12.1998**
(21) Anmeldenummer: 97250190.2
(22) Anmeldetag: 20.06.1997
(51) Int. Cl.: A61K 49/04, A61K 49/00, A61B 6/00

(54) **Verwendung von intravenösen Kontrastmitteln sowie Vorrichtungen für die Projektionsmammographie**

(71) Anmelder: SCHERING AKTIENGESELLSCHAFT, 13353 Berlin (DE)
(72) Erfinder: Speck, Ulrich, Professor Dr., 13465 Berlin (DE)

(57) **Zusammenfassung**

Die Erfindung betrifft die Verwendung von intravenösen Kontrastmitteln für die Projektionsmammographie sowie neue Vorrichtungen für die Projektionsmammographie.

Die Erfindung betrifft daher die Verwendung von intravenösen Kontrastmitteln zur Herstellung eines diagnostischen Mittels für die Projektionsmammographie. Durch die zusätzliche intravenöse Kontrastmittelgabe erreicht die Projektionsmammographie eine mit den modernsten Verfahren wie der Magnetresonanztomographie (MRT) vergleichbare Empfindlichkeit bei deutlich vielseitigerer Einsetzbarkeit und unter Vermeidung der Kosten der MRT. Das neue Verfahren ist einfach und ohne besondere Belastung der Patientinnen durchzuführen und ergibt eine wesentliche Verbesserung
a) der Sensitivität für den Nachweis fokaler Läsionen in der Brust und
b) zusätzliche Informationen über den Charakter vorher erkannter Läsionen.

## Beschreibung

Die Erfindung betrifft die Verwendung von intravenösen Kontrastmitteln für die Projektionsmammographie sowie neue Vorrichtungen für die Projektionsmammographie.

### Stand der Technik

Die Mammographie ist eine seit Jahrzehnten etablierte und immer weiter verbesserte Röntgentechnik zur Früherkennung, zum röntgenologisohen Nachweis, zur Charakterisierung und Lokalisation von Brusttumoren. Sie ist in mehrerer Hinsicht unerreicht in ihrer Leistungsfähigkeit und Verfügbarkeit für die Patientinnen Der größte Nachteil ist eine unvollkommene Nachweisempfindlichkeit für Tumoren geringerer Größe und ohne erkennbaren Mikrokalk.

Es ist schon früh versucht worden, Kontrastmittel zur Verbesserung der Projektionsmammographie einzusetzen. Zu diesem Zweck wurden geeignete Präparate in die Milchgänge eingeführt und deren Verteilung in der Brust zum Nachweis und zur Charakterisierung von Läsionen genutzt. Eine Übersicht gibt die Arbeit von R. Bjørn-Hansen: Contrast-mammography, Brit. J. Radiol 38, 947-951, 1965. Die Technik ist auch als Galaktographie bekannt. Der Kontrast wird durch konzentrierte iodhaltige Kontrastmittel erzielt (> 100 mg Iod/ml). Weiterhin wurden Kontrastmittel direkt in verdächtige oder tumoröse Läsionen der Brust injiziert, um diese entweder zu charakterisieren (z. B. Lehto M. u. Mathiesen T.I.: Adenography: An ancillary diagnostic method of circumscribed lesions of the breast with a positive contrast agent, Breast Dis, 6, 259-268, 1993) oder zu markieren (z. B. Raininko R., Linna M.I., Rasanen O.: Preoperative localization of nonpalpable breast tumors. Acta Chir Scand 142, 575-578, 1976). In beiden Fällen werden unverdünnte, handelsübliche Kontrastmittel direkt zur Darstellung gebracht.

Die intravenöse Gabe von Röntgenkontrastmitteln zur Darstellung parenchymatöser Prozesse in der Projektionsradiographie ist die sehr seltene Ausnahme. Sie gelingt nur, wenn ein Gewebe oder Organ das Kontrastmittel aktiv anreichert. Dafür gibt es bisher zwei Beispiele: Die Darstellung des gesunden Nierenparenchyms durch die heute gebräuchlichen Urographica und die Darstellung des gesunden Leber- und Milzparenchyms durch Emulsionen oder Suspensionen röntgendichter Substanzen. Beide Methoden werden nicht mehr (Leber, Milz) oder nur noch in Ausnahmefällen (Niere) angewandt. Nie ist es gelungen, intravenös verabreichte Röntgenkontrastmittel zur direkten Kontrastierung von Tumoren relevanter Größe in der Projektionsradiographie zu nutzen.

Die Computertomographie und insbesondere die Magnetresonanztomographie sind für ihre sehr viel höhere Meßempfindlichkeit für Kontrastmittel bekannt. Dennoch war es überraschend, daß mit beiden Techniken Brusttumoren nach intravenöser Kontrastmittelinjektion mit großer Sicherheit nachgewiesen werden konnten (Gisvold J.J., Karsell P.R., Reese E.C.: Clinical evaluation of computerized tomographic mammography. Mayo Clin Proc 52, 181-185, 1977; Teifke A., Schweden F., Cagil H., Kanczor H.U., Mohr W., Thelen M.: Spiral-Computertomographie der Mamma. Fortschr Röntgenstr 161, 495-500, 1994; Heywang S.H., Hahn D., Schmidt H., Krischke I., Eiermann W., Bassermann R., Lissner J.: MR imaging of the breast using Gadolinium DTPA. J Comp Ass Tomogr 10, 199-204, 1986.

Auch nach Publikation der Kontrastverstärkung von Brusttumoren durch intravenöse Kontrastmittelgabe in der CT wurde bisher die Nachweisempfindlichkeit der Projektionsmammographie für iodhaltige Kontrastmittel für zu gering gehalten, um diesen in der CT erkennbaren Effekt in der Mammographie nutzen zu können. Die Nutzbarkeit der als weniger röntgendicht bekannten bromhaltigen Kontrastmittel oder der nur in geringerer Konzentration verlügbaren Metallchelatlösungen für diese Anwendung ist daher noch unwahrscheinlicher. Fritz S.L., Chang C.H.J. und Livingston W.H.: (Scatter/primary ratios for X-ray spectra modified to enhance iodine contrast in screen-film mammography, Med Phys 10, 866-870, 1983) untersuchen daher die Frage, ob durch verschiedene physikalische Maßnahmen eine Strahlenqualität erzeugt werden kann, die dem Absorptionsspektrum des Iods besser gerecht wird. Die Ergebnisse ihrer Arbeit werden als noch nicht befriedigend angesehen, einer weiteren Optimierung des Röntgenspektrums werden jedoch gewisse Chancen eingeräumt.

Mitte der achtziger Jahre wurde versucht, die Digitale Subtraktionsangiographie (DSA) mit intravenöser Injektion von Kontrastmitteln einzusetzen. Das Verfahren hat sich nicht durchgesetzt, da die Zuverlässigkeit und Empfindlichkeit zu gering ist und es in jedem Falle eine zusätzliche Untersuchung erfordert (Dean P.B., Sickles E.A.: Invest Radiol 20, 698-699, 1985).

Die genannten Methoden haben Vorteile gegenüber der konventionellen Projektionsmammographie, aber auch bedeutende Nachteile wie hohe Kosten und beschränkte Verfüngbarkeit, fehlender Nachweis des für die Tumordiagnostik wichtigen Mikrokalk, geringere räumliche Auflösung, lange Untersuchungsdauer, schwierige Zugänglichkeit für Biopsien bzw. höhere Strahlenexposition. Wenn auch nicht jeder Nachteil für jede Technik zutrifft, werden MR und erst recht CT heute nur bei einem sehr geringen Teil der betreffenden Patientinnen eingesetzt, die DSA wird praktisch überhaupt nicht für den Nachweis von Brusttumoren verwandt.

Wegen der nahezu universellen Verfügbarkeit, geringen Kosten und in vieler Hinsicht hohen Leistungsfähigkeit, ist daher eine Verbesserung der eingeführten Projektionsmammographie im Hinblick auf einen sicheren Tumornachweis von großer Bedeutung. In dieser Hinsicht sind auch bereits viele Versuche unternommen worden. Insbesondere wurden die Aufnahmetechnik und das verwendete Filmmaterial über Jahrzehnte optimiert; die Xeroradiographie wurde erprobt. Neue Empfängersysteme und die Digitalisierung versprechen weitere Fortschritte. Dennoch liegt die Projektiortsmammographie, soweit bisher absehbar, deutlich unter der Sensitivität der bisher besten Methode, der kontrastverstärkten Magnetresonanztomographie.

### Beschreibung der Erfindung

Es wurde nun völlig überraschend gefunden, daß sich die als recht kontrastmittelunempfindlich bekannte Projektionsradiographie im speziellen Falle der Projektionsmammographie durch intravenöse Kontrastmittelgabe verbessern läßt, obwohl die Kontrastmittel auf dem Weg durch Herz und Lunge sehr stark verdünnt werden und eine aktive Anreicherung in Brusttumoren nicht bekannt ist.

Die Erfindung betrifft daher die Verwendung von intravenösen Kontrastmitteln zur Herstellung eines diagnostischen Mittels für die Projektionsmammographie.
Durch die zusätzliche intravenöse Kontrastmittelgabe erreicht die Projektionsmammographie eine mit den modernsten Verfahren wie der Magnetresonanztomographie (MRT) vergleichbare Empfindlichkeit bei deutlich vielseitigerer Einsetzbarkeit und unter Vermeidung der Kosten der MRT. Das neue Verfahren ist einfach und ohne besondere Belastung der Patientinnen durchzuführen und ergibt eine wesentliche Verbesserung
a) der Sensitivität für den Nachweis fokaler Läsionen in der Brust und
b) zusätzliche Informationen über den Charakter vorher erkannter Läsionen.

Die erfindungsgemäße Verwendung kann mit heute verfügbaren Geräten und Mitteln z. B. wie folgt durchgeführt werden, sofern die Geräte bei niedriger Strahlenenergie - wie in der Projektionsmammographie üblich - betrieben werden.

Das Meßverfahren wird vorzugsweise wie folgt durchgeführt:
1) Es wird ein normales Mammogramm aufgenommen (Prä-Kontrastaufnahme).
2) Die Patientin erhält ein gebräuchliches urographisches Röntgenkontrastmittel in einer Dosis von zirka 0,5 g bis 1,5g Iod/kg Körpergewicht rasch intravenös injiziert oder infundiert.
3) 30 Sekunden bis 1 Minute nach Ende der Injektion wird ein zweites Mammogramm aufgenommen (Post-Kontrastaufnahme). Gegebenenfalls werden weitere Aufnahmen bis zirka 5 Minuten nach Injektionsende aufgenommen, welche bei Bedarf zusätzliche Informationen über die Eigenschaften der Läsion geben können.

Geräte und Geräteeinstellungen von weniger als 50 kV sind für die erfindungsgemäße Verwendung geeignet; bevorzugt ist die Nutzung von Strahlung entsprechend 20 kV bis 40 kV, besonders bevorzugt ist eine Strahlenenergie von 25 kV bis 35 kV.

Für die erfindungsgemäße Verwendung sind alle Verbindungen geeignet, die für die Herstellung wasserlöslicher urographischer Kontrastmittel üblicherweise Verwendung finden. Als Beispiele seien genannt: Meglumin oder Lysin Diatrizoat, Iothalamat, Ioxithalamat, Iopromid, Iohexol, Iomeprol, Iopamidol, Ioversol, Iobitridol, Iopentol, Iotrolan, Iodixanol und Ioxilan (INN).

Es können aber auch iodfreie Verbindungen verwendet werden, wie z.B.:
1. Kontrastmittel, die Brom als bildgebendes Element enthalten,
2. Kontrastmittel, die Elemente der Ordnungszahlen 34, 42, 44 - 52, 54 - 60, 62 - 79, 82 oder 83 als bildgebendes Element enthalten,
3. Kontrastmittel, die Chelatverbindungen von Elementen der Ordnungszahlen 56 - 60, 62 - 79, 82 oder 83 als bildgebendes Element enthalten.

Die Erfindung betrifft daher auch die Verwendung derartiger iodfreier Verbindungen.

Die heute gebräuchlichen urographischen Röntgenkontrastmittel sind für das beschriebene Verfahren hervorragend geeignet. Es wurde überraschend gefunden, daß anders als bei nahezu jedem anderen Röntgenverfahren in der Projektionsmammographie das Element Iod gegen das Element Brom ganz oder teilweise ausgetauscht werden kann. Dies ist zwar in der Vergangenheit auch diskutiert worden, hat sich aber wegen der deutlich geringeren Strahlenabsorption von Brom gegenüber Iod in keinem Röntgenverfahren bewährt. Die Projektionsmammographie stellt insofern eine Ausnahme dar. Sie ist eine neuartige überraschende Anwendung für die z.B. in der EP 0 118 348 A1 beschriebenen Verbindungen.

Weiterhin sind ausscheidbare und verträgliche Kontrastmittel basierend auf anderen kontrastgebenden Elementen, molekularen und supramolekularen Strukturen ebenfalls für die erfindungsgemäße Verwendung geeignet.

Als kontrastgebende Elementen sind vor allem solche mit den Ordnungszahlen 34, 42, 44 - 60, 62 - 79, 82 oder 83 geeignet. Die kontrastgebenden Elemente können kovalent an organische Moleküle gebunden sein oder als Komplexe vorliegen oder in makromolekulare Strukturen integriert sein. Besonders vorteilhaft sind Substanzen mit einem Molekulargewicht von 10 000 bis 80 000 D. Weiterhin können die einzelnen Kontrastmittelmoleküle Bestandteil größerer Strukturen wie Assoziate, Liposomen, Emulsionströpfchen und Mikro- bzw. Nanopartikel sein (Parvez Z., Moncada R., Sovak M., edts.: Contrast Media: Biological Effects and clinical application. Vol. III, CRC Press, Boca Raton, Florida 1987, 73-130).

Die Zubereitung erfolgt in pharmazeutisch üblicher Form in physiologisch verträglichen Trägermedien, bevorzugt Wasser, unter Verwendung gebräuchlicher Hilfsstoffe wie Stabilisatoren (z. B. Komplexe, Komplexbildner, Antioxydantien), Puffer (z. B. Tris, Citrat, Bicarbonat), Emulgatoren und Substanzen zur Anpassung der Osmolalität und des Elektrolytgehaltes je nach Bedarf.

Bevorzugt sind Kontrastmittel mit Konzentrationen von 100 mg Iod/ml bis 500 mg Iod/ml, besonders bevorzugt sind nichtionische Röntgenkontrastmittel mit 200 mg Iod/ml bis 400 mg Iod/ml oder einer entsprechenden Röntgendichte bei Wahl eines anderen strahlenabsorbierenden Elementes. Das Mittel kann in einer Dosis von 150 bis 1500 mg Iod/kg Körpergewicht (KG) appliziert werden.

Bei der erfindungsgemäßen Verwendung bromhaltiger Verbindungen wird im Kontrastmittel eine Konzentration von 100 bis 500 mg Brom/ml bevorzugt. Die applizierbare Dosis beträgt 100 bis 1500 mg Brom/kg Körpergewicht.

Bei der erfindungsgemäßen Verwendung von Verbindungen der Elemente der Ordnungszahlen 34, 42, 44 - 52, 54 - 60, 62 - 79, 82 oder 83 wird im Kontrastmittel eine Konzentration von 10 mmol bis 2 mol/l - bezogen auf das bildgebende Element - bevorzugt. Die applizierbare Dosis beträgt 0,1 bis 2 mmol /kg Körpergewicht (bezogen auf das bildgebende Element). Bevorzugt ist der Bereich 0,2 bis 0,6 mmol/kg Körpergewicht.

Bei der erfindungsgemäßen Verwendung von die Chelatverbindungen von Elementen der Ordnungszahlen 56 - 60, 62 - 79, 82 oder 83 wird im Kontrastmittel eine Konzentration von 10 mmol bis 2 mol/l - bezogen auf das bildgebende Element - bevorzugt. Die applizierbare Dosis beträgt 0,1 bis 2 mmol/kg Körpergewicht (bezogen auf das bildgebende Element). Bevorzugt ist der Bereich 0,2 bis 0,6 mmol/ kg Körpergewicht.

Eine bei der erfindungsgemäßen Verwendung sehr vorteilhafte Variante der intravenösen Kontrast-Projektionsmammographie betrifft die Nutzung der Subtraktionstechnik, die in der Projektionsmammographie bisher nicht eingeführt ist. Entsprechende Verfahren haben sich aber in der Angiographie sehr bewährt. In der Angiographie sind allerdings auch wiederum wesentlich höhere lokale Iodkonzentrationen (im Blut) erforderlich, als sie in Brusttumoren erreichbar sind. Insofern war die Möglichkeit des Einsatzes dieser Technik zum Nachweis kleiner Läsionen nicht vorhersehbar. Das Verfahren beruht im wesentlichen auf folgenden Schritten:
1) Es wir ein normales Mammogramm aufgenommen (Prä-Kontrastaufnahme). Die Daten werden gespeichert.
2) Die Patientin erhält ein geeignetes Kontrastmittel in ausreichender Dosis rasch intravenös injiziert.
3) Ab 30 Sekunden nach Injektionsende werden eine oder mehrere weitere Mammogramme aufgenommen und gespeichert.
4) Die unter (1) erhobenen Daten werden mit den unter (3) erhobenen Daten korreliert (vorzugsweise subtrahiert) und das Ergebnis entsprechend verstärkt und als Bild ausgegeben.
5) Gegebenenfalls werden Daten zur Geschwindigkeit und zum Ausmaß des Kontrastmittelanstiegs und zur Kinetik des Auswaschvorganges errechnet und separat dargestellt.

Die Erfindung betrifft daher auch eine Vorrichtung zur Projektionsmammographie gekennzeichnet durch mindestens eine Speichervorrichtung für die Prä-Kontrastaufnahme, mindestens eine Speichervorrichtung für die Post-Kontrastaufnahme, mindestens eine Recheneinheit zu Korrelation (insbesondere Subtraktion) der verschiedenen Aufnahmen und eine Ausgabevorrichtung für das berechnete Mammogramm.

Außer der Korrelation von zeitlich aufeinanderfolgenden Aufnahmen oder Datensätzen ist auch die Korrelation von Aufnahmen, die mit unterschiedlicher Strahlenenergie angefertigt wurden von Vorteil. So kann z.B. bei der erfindungsgemäßen Verwendung bromhaltiger Verbindungen eine Aufnahme mit einer Strahlenenergie von ε₁=35kV und eine Aufnahme mit einer Strahlenenergie von ε₂=25kV durchgeführt und die gespeicherten Aufnahmen miteinander korrelliert - insbesondere voneinander subtrahiert - werden. In diesem Fall wird ebenfalls eine Unterdrückung der normalen Gewebestrukturen zugunsten des kontrastgebenden, intravenös zugeführten Elementes erreicht, da sich die Strahlenabsorption des Gewebes bei den gewählten Energien von denjenigen des Kontrastmittels unterscheidet. Durch wiederholte Messung kann auch mittels einer derartigen Vorrichtung der zeitliche Verlauf der Kontrastmittelkonzentration erfaßt und ausgewertet werden.

Ein weiterer Gegenstand der Erfindung ist daher eine Vorrichtung zur Projektionsmammographie gekennzeichnet durch mindestens eine Speichervorrichtung für eine Aufnahme bei einer Strahlenenergie ε₁, mindestens eine Speichervorrichtung für eine Aufnahme bei einer Strahlenenergie ε₂, mindestens eine Recheneinheit zur Korrelation der verschiedenen Aufnahmen und eine Ausgabevorrichtung für das berechnete Mammogramm.

Bei der klassischen Projektionsmammographie wird jeweils nur eine Mamma untersucht. Zur Limitierung der nötigen Kontrastmittelmenge ist es bei der erfindungsgemäßen Verwendung vorteilhaft, beide Mammae simultan zu untersuchen. Vorrichtungen, die eine derartige Untersuchung erlauben, sind bisher nicht bekannt. Gegenstand der Erfindung sind daher auch Vorrichtungen, die dadurch gekennzeichnet sind, daß sie die simultane Untersuchung beider Mammae ermöglichen.

### Ausführungsbeispiele:

Die folgenden Beispiele sollen den Erfindungsgegenstand erläutern ohne ihn auf diese beschränken zu wollen.

### Beispiel 1: Phantomstudien

Wismut-, Iod- und bromhaltige Kontrastmittellösungen ((4S)-4-(Ethoxybenzyl)3,6,9-tris(carboxylatomethyl)-3,6,9-triazaundecansäure, Wismutkomplex, Dinatriumalz, Iotrolan (INN) bzw. N-Cetyl-N,N,N-Trimethylammoniumbromid) werden mit einer Konzentration von 9,8 mg Bi/ml, 6 mg Iod/ml, bzw. 3,8 mg Br/ml in 2% Agar hergestellt. Die Agargele werden in Schichtdicken von 3 mm, 5 mm oder 10 mm geschnitten. Die kontrastmittelhaltigen Gele sowie ein Kontrollgel mit 2,8 mg NaCl/ml werden in einen Agarblock von 5 cm Stärke integriert. Das gesamte Phantom wird bei 28 kV und 63 mA entsprechend einem Mammogramm geröntgt, wobei die Röntgenstrahlung jeweils zirka 4 cm bis 5 cm kontrastmittelfreien Agar zu passieren hat und 3 mm bis 10 mm kontrastmittelhaltigen Agar.

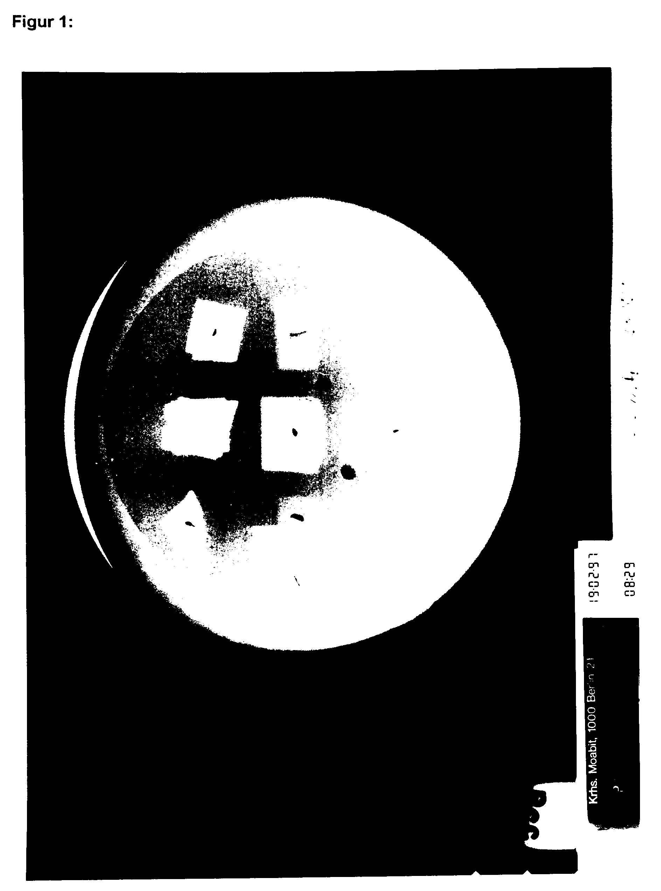

Ergebnis: Selbst die nur ca. 3 mm starken kontrastmittelhaltigen Agarstücke sind gut erkennbar. Brom ist bei äquimolarer Konzentration überaschenderweise etwa doppelt so wirksam wie Iod; Wismut mehr als dreifach so wirksam wie Iod (Figur 1).

Die Figur 1 zeigt eine Röntgenaufnahme bei 28 kV, 63 mA eines Agarphantoms mit eingebetteten kontrastmittelhaltigen Agarblöcken von: linke Reihe 5 mm Dicke, mittlere Reihe 10 mm Dicke, reche Reihe 3 mm Dicke. Die Blöcke der oberen Reihe enthalten 3,8 mg Brom/ml, die der mittleren Reihe 6 mg Iod/ml, die der unteren Reihe 9,8 mg Bi/ml.

Der Block mit NaCl ist nicht sichtbar.

### Beispiel 2: Intravenöse Kontrastmittel-Mammographie

Bei einer Patientin wurde mammographisch ein 1,5 cm x 0,8 cm großes Mammakarzinom aufgrund von Strukturen, Mikrokalk und Biopsie nachgewiesen. Präoperativ ist auf Multifocalität zu prüfen; dafür wird der Patientin eine 1er-Verweilkanüle in die linke Armvene gelegt (V. cubitalis). Die Projektionsmammographie wird vor Kontrastmittelgabe wiederholt. Unmittelbar nach der Nativaufnahme beginnt die Infusion von 3 ml/kg Ultravist®-300 (Schering AG, Berlin; Wirkstoff: Iopromid (INN)) mit einer Geschwindigkeit von 3 ml/sec. mittels eines automatischen Injektors. Die erste Aufnahme nach Kontrastmittelgabe erfolgt 1 Minute nach Infusionsende. Die Position von Patientin und Aufnahmegerät bleibt während dieser Zeit völlig unverändert, ebenso die Aufnahmebedingungen mit 28 kV Röhrenspannung und 63 mAs.

Die Aufnahmen nach Kontrastmittelinjektion zeigen einen wesentlich vergrößerten Bereich der Kontrastmittelaufnahme gegenüber dem als Tumorbereich definierten Gewebe vor Kontrastmittelgabe, jedoch keine weiteren separaten anreichernden Herde in der Brust.

## Patentansprüche

1. Verwendung von intravenösen Kontrastmitteln zur Herstellung eines diagnostischen Mittels für die Projektionsmammographie.

2. Verwendung eines Mittels nach Anspruch 1, dadurch gekennzeichnet, daß das intravenöse Kontrastmittel Iod als kontrastgebendes Element enthält.

3. Verwendung eines Mittels nach Anspruch 1, dadurch gekennzeichnet, daß das intravenöse Kontrastmittel Brom als kontrastgebendes Element enthält.

4. Verwendung eines Mittels nach Anspruch 1, dadurch gekennzeichnet, daß das intravenöse Kontrastmittel eine Verbindung der Elemente der Ordnungszahlen 34, 42, 44 - 52, 54 - 60, 62-79, 82 oder 83 enthält.

5. Verwendung eines Mittels nach Anspruch 1, dadurch gekennzeichnet, daß das intravenöse Kontrastmittel ein Metallchelat der Elemente der Ordnungszahlen 56-60, 62 - 79, 82 oder 83 enthält.

6. Verwendung eines Mittels nach Anspruch 1, dadurch gekennzeichnet, daß das intravenöse Kontrastmittel ein Molekulargewicht von 10.000 bis 80.000 D aufweist.

7. Verwendung eines Mittels nach Anspruch 1, dadurch gekennzeichnet, daß das intravenöse Kontrastmittel in höhermolekularen Strukturen vorliegt.

8. Verwendung eines Mittels nach Anspruch 7, dadurch gekennzeichnet, daß das intravenöse Kontrastmittel in Form von Molekülassoziaten, Liposomen, Nano- oder Mikropartikeln vorliegt.

9. Verwendung von intravenösen Kontrastmitteln nach Anspruch 1, dadurch gekennzeichnet, daß sie in einer Röntgendichte vorliegen, die 100 mg Iod/ml bis 500 mg Iod/ml entspricht.

10. Verwendung von intravenösen Kontrastmitteln nach Anspruch 2, dadurch gekennzeichnet, daß sie in einer Konzentration von 100 mg Iod/ml bis 500 mg Iod/ml vorliegen.

11. Verwendung von intravenösen Kontrastmitteln nach Anspruch 2, dadurch gekennzeichnet, daß sie in einer Dosis entsprechend 150 mg Iod/kg bis 1500 mg Iod/kg Körpergewicht verabreicht werden.

12. Verwendung von intravenösen Kontrastmitteln nach Anspruch 3, dadurch gekennzeichnet, daß sie in einer Konzentration von 100 mg Brom/ml bis 500 mg Brom/ml vorliegen.

13. Verwendung von intravenösen Kontrastmitteln nach Anspruch 3, dadurch gekennzeichnet, daß sie in einer Dosis entsprechend 100 mg Brom/kg bis 1500 mg Brom/kg Körpergewicht verabreicht werden.

14. Verwendung von intravenösen Kontrastmitteln nach Anspruch 4, dadurch gekennzeichnet, daß sie in einer Konzentration von 10 mmol - 2 mol/l vorliegen.

15. Verwendung von intravenösen Kontrastmitteln nach Anspruch 4, dadurch gekennzeichnet, daß sie in einer Dosis von 0,1 - 2 mmol/kg Körpergewicht appliziert werden.

16. Verwendung von intravenösen Kontrastmitteln nach Anspruch 5, dadurch gekennzeichnet, daß sie in einer Konzentration von 10 mmol/l - 2 mol/l vorliegen.

17. Verwendung von intravenösen Kontrastmitteln nach Anspruch 5, dadurch gekennzeichnet, daß sie in einer Dosis von 0,1 - 2 mmol/kg Körpergewicht appliziert werden.

18. Vorrichtung zur Projektionsmammographie gekennzeichnet durch mindestens eine Speichervorrichtung für die Prä-Kontrastaufnahme, mindestens eine Speichervorrichtung für die Post-Kontrastaufnahme, mindestens eine Recheneinheit zur Korrelation der verschiedenen Aufnahmen und eine Ausgabevorrichtung.

19. Vorrichtung zur Projektionsmammographie gekennzeichnet durch mindestens eine Speichervorrichtung für eine Aufnahme bei einer Strahlenenergie ε₁, mindestens eine Speichervorrichtung für eine Aufnahme bei einer Strahlenenergie ε₂, mindestens eine Recheneinheit zur Korrelation der verschiedenen Aufnahmen und eine Ausgabevorrichtung.

20. Vorrichtung nach Anspruch 18, dadurch gekennzeichnet, daß die Projektionsmammographie bei weniger als 50 kV durchgeführt wird.

21. Vorrichtung nach Anspruch 19, dadurch gekennzeichnet, daß mindestens eine der Strahlenenergien ε₁ und ε₂ weniger als 50 kV beträgt.

22. Vorrichtung nach Anspruch 18 oder 19, dadurch gekennzeichnet, daß sie die simultane Untersuchung beider Mammae ermöglicht.

23. Vorrichtung nach Anspruch 18 oder 19, dadurch gekennzeichnet, daß sie den zeitlichen Verlauf der kontrastmittelbedingten Strahlenabsorption erfaßt.
